(19) Europäisches Patentamt
European Patent Office
Office européen des brevets

(11) Numéro de publication: **0 007 879**
**B1**

(12) **FASCICULE DE BREVET EUROPEEN**

(45) Date de publication du fascicule de brevet: **25.11.81**

(51) Int. Cl.³: **C 12 Q 1/00, G 01 N 33/54**

(21) Numéro de dépôt: **79400542.1**

(22) Date de dépôt: **31.07.79**

(54) **Delta-5,3-céto stéroide isomérase et son application dans les dosages immunoenzymatiques.**

(30) Priorité: **02.08.78 FR 7822867**

(43) Date de publication de la demande:
**06.02.80 Bulletin 80/3**

(45) Mention de la délivrance du brevet:
**25.11.81 Bulletin 81/47**

(84) Etats Contractants Désignés:
**AT BE CH DE GB IT LU NL SE**

(56) Documents cités:
**FR - A - 2 382 695**
**US - A - 4 067 774**

(73) Titulaire: **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur: **Nicolas, Jean-Claude**
**Le Phaéton Le Cervantès Avenue du père Soulas**
**F-34000 Montpellier (FR)**
Inventeur: **Terouanne, Béatrice**
**10, rue Alauzet**
**F-34000 Montpellier (FR)**
Inventeur: **Descomps, Bernard**
**26, Avenue d'Assas**
**F-34000 Montpellier (FR)**
Inventeur: **Crastes de Paulet, André**
**12, Avenue Lepic**
**F-34000 Montpellier (FR)**

(74) Mandataire: **Harlé, Robert et al,**
***c/o* Cabinet Harlé & Lechopiez 21, rue de la Rochefoucauld**
**F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 007 879 B1

Delta-5,3-céto stéroide isomérase et son application dans les dosages immunoenzymatiques

La présente invention concerne le domaine des dosages immunoenzymatiques.

On connaît depuis longtemps des procédés de dosage radioimmunologiques pour la détermination quantitative de la concentration dans des milieux biologiques de certaines substances, en particulier des antigènes ou des anticorps ainsi que de certaines hormones. Toutefois, les méthodes de dosage radio-immunologiques présentent des inconvénients. Parmi ceux-ci,il faut souligner les contraintes de mise en oeuvre qui sont entraînées par l'utilisation de marqueurs radioactifs, ce qui ne permet pas de rendre la méthode accessible à n'importe quel laboratoir.

Au cours des dix dernières années, les progrès importants accomplis dans l'enzymologie ont permis de substituer aux dosages radio-immunologiques des dosages immunoenzymatiques faisant appel à une enzyme comme marqueur au lieu des marqueurs radioactifs utilisés jusqu'alors. On connaît de nombreuses références bibliographiques dans le domaine des dosages immunoenzymatiques; il suffira de se reporter par exemple à l'article de S. AVRAMEAS "Détection d'anticorps et d'antigènes à l'aide d'enzymes" Bull. Soc. Chim. Biol. 1968, *50*, N° 5—6, ainsi qu'aux brevets français n° 74.34.519 et 75.36.889.

Il existe un certain nombre de techniques de dosage immunoenzymatique.

Par exemple, si la technique est utilisée pour doser des anticorps, un mode de mise en oeuvre consiste:

— à faire incuber les anticorps à doser avec un antigène insolublisé,
— à laver la phase solide après élimination des surnageants,
— à faire incuber la phase solide avec un anticorps marqué à l'aide d'une enzyme et
— à mesurer l'activité enzymatique de la phase solide.

Dans une telle technique, on peut utiliser un support insoluble quelconque sur lequel est fixé l'anticorps par des moyens connus.

Dans une autre technique, dite de "dosage sandwich", appliquée par exemple au dosage d'anticorps, on procède à la succession d'opérations ci-après:

— faire incuber les anticorps à tester avec un antigène rendu insoluble,
— laver la phase solide après élimination des surnageants, et
— incuber cette phase solide avec un antigène couplé avec une enzyme.

Il est connu également que les mesures peuvent être réalisées à l'aide des mêmes techniques non pas dans la phase solide mais dans la phase liquide. Dans tous les cas, c'est l'activité enzymatique de la phase choisie que est le paramètre déterminant du dosage.

Toutes les indications ci-dessus sont bien connues de l'homme de l'art et n'ont été donées que pour illustrer le domaine auquel est applicable la présente invention.

La technique de dosage immunoenzymatique a pour avantage de pouvoir être mise en oeuvre sans contrainte particulière, ce qui n'est pas le cas des dosages radio-immunologiques. Cependant, la mise en oeuvre pratique des dosages immunoenzymatiques soulève encore certaines difficultés. En effet, la méthode est limitée quant à sa sensibilité et ne permet pas de détecter des quantités très faibles d'antigène, d'anticorps ou d'hormone dans les milieux biologiques à tester. Il existe donc encore des cas où, à l'heure actuelle, aucune technique de dosage n'est accessible, si la concentration de la substance active à mesurer est trop taible. Il importe également d'apporter des perfectionnements aux techniques de couplage entre les antigènes et les anticorps avec les enzymes respectivement, pour améliorer les rendements de fixation et éviter des réactions secondaires parasites. Enfin, il peut aussi se poser des problèmes de stabilité des marqueurs d'enzyme au cours du temps.

L'invention a pour objet un procédé de dosage immunoenzymatique qui, grâce à l'utilisation à titre de marqueur d'une enzyme particulièrement appropriée, permet d'effectuer des mesures avec des sensibilités jamais atteintes jusqu'alors, ce qui offre la possibilité de réaliser des dosages qui ne pouvaient pas être effectués jusqu'à présent.

Grâce à la mise en oeuvre de ladite enzyme ce dosage immunoenzymatique se prête à une automatisation complète sur des appareils existant déjà et utilisés pour des dosages radio-immunologiques.

Grâce aux propriétés particulières de l'enzyme en cause, le dosage immunoenzymatique, peut être mis en oeuvre sans qu'il soit nécessaire de passer par une étape préalable d'insolubilisation de la substance active à mesurer, par exemple de l'anticorps ou de l'antigène.

Selon un mode particulier de couplage de l'antigène ou de l'anticorps avec l'enzyme considérée, ledit couplage est réalisé d'une manière sélective et avec un rendement élevé.

L'invention a pour objet l'application de la $^{\Delta}$5,3-céto stéroïde isomérase comme marqueur ensymatique dans les dosages immunoenzymatiques, l'activité enzymatique, qui constitue une valeur représentative de la concentration de la substance à mesurer, étant déterminée par une mesure d'absorption dans l'ultraviolet à 248 nm.

La Δ5,3-céto stéroïde isomérase se prête à l'utilisation comme marqueur enzymatique dans toutes les techniques connues actuelles de dosage immunoenzymatique, par exemple les méthodes dites de double anticorps, dans lesquelles l'activité enzymatique est mesurée en solution. Toutefois, ainsi qu'il a été indiqué précédemment, la nature particulière de l'enzyme, dont l'application est proposée par l'invention, permet des modes de mise en oeuvre de la technique de dosage immunoenzymatique qui sont simplifiés et perfectionnés par rapport à ceux qui étaient déjà connus.

En tant que telle, la $\Delta_5$,3-céto stéroïde isomérase est une enzyme connue. A titre de références bibliographiques relatives à cette enzyme, on peut citer par exemple les extraits de l'ouvrage "Methods in Enzymology" vol. 5 (1962) p.527 et suivantes (Academic Press) et Vol. 34 (1974) p.557 et suivantes (William B. Jakoby and Meir Wilchek). La $\Delta_5$,3-céto stéroïde isomérase est une enzyme réputée active et donnant lieu à des réactions rapides. Les effets qu'elle présente dans l'application au dosage immunoenzymatique étaient tout-à-fait imprévisibles. En effet, il n'est pas possible de prévoir à l'avance le comportement d'une enzyme particulière lors de son couplage avec un antigène ou un anticorps. On sait en effet que certaines enzymes ont une tendance à se dénaturer ou à s'inactiver lors de la réaction de couplage. En outre, la structure chimique elle-même de l'enzyme pose un problème particulier, car il s'agit d'une enzyme à site hydrophobe. Certains agents de couplage, couramment utilisés dans la technique immunoenzymatique, tels que le glutaraldéhyde ou le phényl diisocyanate se révèlent moins appropriés aux besoins de l'invention. Il est à noter également que la grande activité de l'enzyme aurait pu rendre impossible l'automatisation de dosage de l'invention, alors que, ainsi qu'on l'a noté précédemment, l'utilisation de la $\Delta_5$,3-céto stéroïde isomérase permet d'automatiser les dosages sur des appareils existant déjà en radio-immunologie.

L'enzyme utilisée selon l'invention est l'isomérase du microorganisme *Pseudomonas Testosteroni*. On a donné dans l'exemple 1 qui suit des indications détaillées sur la culture du microorganisme, la préparation d'une poudre acétonique et la purification de l'enzyme. L'enzyme est purifiée par chromatographie sur DEAE cellulose puis chromatographie d'affinité sur oestradiol-agarose selon la méthode décrite dans Method in Enzymology Vol. 34 (1974) p.557 et suivantes citée supra.

L'activité spécifique de l'enzyme utilisée est élevée, par exemple de l'ordre de 30.000 UI/mg à 60.000 UI/mg.

La $\Delta_5$,3-céto stéroïde isomérase de *P. Testosteroni* possède une très haute activité spécifique et un faible poids moléculaire (25.000 environ):la première propriété permet des dosages de sensibilité jamais atteinte jusqu'ici en immunoenzymologie, la seconde (faible poids moléculaire) permet de réaliser un marquage de l'antigène qui conserve à ce dernier une bonne immunoréactivité.

L'activité de l'enzyme est déterminée selon la technique décrite par TALALAY [J. Biol. Chem. 237 (1962) 1500—1506]. Une unité d'activité correspond à la quantité d'enzyme isomérisant 1 $\mu$ mole de $\Delta_5$-androstène dione par minute à 25°C et à pH = 7. L'absorbance d'l mole/l de $\Delta_4$-androstène dione en solution aqueuse est de 16.300 à 248 nm.

L'absorbance d'l $\mu$mole de $\Delta_4$-androstène dione en solution dans un milieu réactionnel de 1 ml est égale à:

$$\frac{16\ 300 \times 10^3}{1 \times 10^6} = 16,3$$

et l'activité de l'isomérase est donnée par la formule

$$\text{Act U/ml} = D_o \times \frac{1}{16,3} \text{ où}$$

$D_o$ = absorbance mesurée.

Les solutions contenant 1 mg d'isomérase par ml ont une absorbance égale à 0,328 à 280 nm; donc:

$$\text{AS UI/mg} = \frac{D_o}{16,3} \times \frac{0,328}{\text{abs}}$$

1 picomole d'enzyme dans 1 ml donnera par minute à 248 nm une variation d'absorbance de 12 unités d'absorbance, ce qui permet de détecter après 10 minutes d'incubation une fentomole d'enzyme ($D_o$ = 0,120).

Ainsi, la très forte activité spécifique de la $\Delta_5$,3-céto stéroïde isomérase, utilisée comme marqueur dans les dosages immunoenzymatriques, permet de détecter des quantités d'antigène de l'ordre de la fentomole. On donnera ci-après en détail un exemple particulier d'application au dosage de l'hormone lactogène placentaire HLP. Les résultats obtenus montrent que l'antigène marqué par l'isomérase présente une immunoréactivité du même ordre de grandeur que celle d'une hormone radioactive

utilisée dans un dosage radioimmunologique.

L'enzyme proposée par l'invention présente un grand intérêt pour les dosages immunoenzymatiques des substances à très faible concentration éventuellement présentes dans des milieux biologiques, telles que les hormones hypophysaires et les stéroïdes plasmatiques.

Bien entendu, l'invention n'est pas limitée aux exemples d'application qui viennent d'être décrits. En effet, l'invention s'applique à la détection et à la détermination de toutes sortes de protéines fixantes et de substances conjuguées correspondantes.

On peut réaliser un mode de couplage de l'enzyme avec l'antigène qui peut être de nature différente selon la nature de l'antigène, par exemple selon qu'il s'agit d'une hormone peptidique ou d'une hormone stéroïde. Dans tous les cas, cependant, le couplage fait intervenir un résidu thiol de l'enzyme.

Ainsi, dans le cas du couplage de la $^{\Delta}5$,3-céto stéroïde isomérase avec une hormone stéroïde, celle-ci, désignée par l'abréviation Ag, est mise à réagir avec l'acide bromoacétique de formule $Br—CH_2—COOH$ en présence de carbodiimide. Par ailleurs, l'enzyme est transformée en une enzyme portant des groupes —SH par réaction avec de l'anhydride S-acétyl-mercapto succinique. Finalement l'hormone portant le substituant bromo est mise à réagir avec l'enzyme portant les groupements —SH, l'ensemble du couplage s'effectuant conformément au schéma réactionnel ci-après:

$$Ag + Br - CH_2—COOH \xrightarrow{\text{carbodiimide}} Ag—COO—CH_2—Br$$

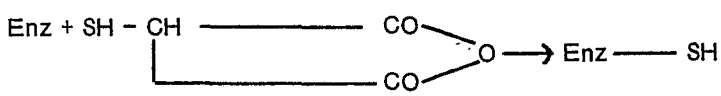

$$Ag - COO—CH_2—Br + Enz - SH \longrightarrow Ag—COO—CH_2—S—Enz.$$

La dernière réaction étant pratiquement quantitative, le couplage réalisé est très satisfaisant.

Le nombre de molécules d'antigène liées par molécule d'enzyme peut être facilement déterminé par spectrophotométrie. Cette méthode permet de contrôler efficacement la liaison des molécules d'antigène à l'enzyme. Dans le cas de la progestérone, on peut introduire 2 à 4 molécules de stéroïde par molécule d'enzyme.

Selon un mode de réalisation particulièrement avantageux de l'invention, il est proposé un mode de couplage spécial dans le cas des hormones peptidiques. Dans ce cas, en effet, le couplage avec l'hormone peptidique est réalisé après introduction de résidus thiols avec formation d'un pont disulfure. L'exemple 2 qui suit illustrera l'application de ce mode de couplage à l'hormone lactogène placentaire HLP.

L'amidination de l'HLP par le 4-mercaptobutyrimidate de méthyle permet d'introduire deux résidus thiols par mole d'hormone.

L'anhydride S-acétyl mercaptosuccinique permet d'introduire 9 résidus thiols par mole d'enzyme.

L'incubation de 0,1 $\mu$m d'HLP contenant 2 résidus thiols par mole avec 0,05 $\mu$m d'isomérase contenant 9 groupements disulfures mixtes (dithio-5 (2-nitrobenzoate)succinamide) conduit à la libération de 0,1 $\mu$m de thionitrobenzoate. La réaction est complète au bout de 4 heures. L'activité enzymatique mesurée à ce stade montre que 80% de l'activité enzymatique de départ sont conservés.

D'une manière générale, le mode de couplage proposé par l'invention peut être représenté par le schéma réactionnel ci-après.

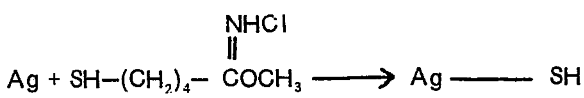

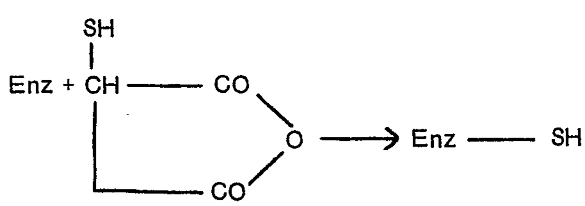

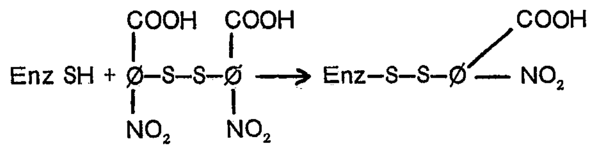

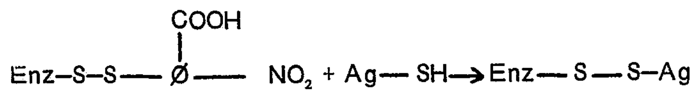

Le mode de couplage proposé par l'invention présente l'avantage de ne pas donner lieu à la formation de hauts polymères enzyme-antigène; le rendement de couplage est élevé, même pour des concentrations en protéines faibles (0,2 mg/ml). L'enzyme peut être libérée du complexe antigène-anticorps et son activité enzymatique peut être ainsi mesurée en phase homogène, ce qui permet d'augmenter la précision de la mesure. Pour libérer l'enzyme, il suffit de faire agir sur le complexe antigène-anticorps, porteur de l'enzyme, un réducteur, tel que le dithiotreitol ou le $\beta$-mercaptoéthanol.

Le couplage antigène-enzyme est stable dans les conditions normales. Il n'y a pas de libération par les protéines sériques. En outre, le couplage est stoechiométrique:une mole d'antigène est liée à une mole d'enzyme. On a constaté que l'enzyme couplée à l'antigène est stable pendant au moins 1 an à 4°C.

On a procédé à des essais de couplage pour comparer les résultats obtenus avec divers réactifs connus de couplage et avec les réactifs selon l'invention qui permettent de réaliser une liaison par un pont disulfure. Dans tous les essais on a utilisé la $^{\Delta}$5,3-céto stéroïde isomérase comme enzyme pour le couplage. On a ensuite mesuré le rendement de couplage, c'est-à-dire le pourcentage de produit couplé à l'isomérase, ainsi que l'activité résiduelle enzymatique du produit couplé. Les réactifs connus utilisés à titre de comparaison ont été:le glutaraldéhyde, (à des concentrations de 2% et 0,02% en poids) le phényl diisocyanate (à pH 7 et à pH 5) et le diméthylsuberimidate.

Les essais de couplage de la $^{\Delta}$5-3-céto stéroïde isomérase avec l'hormone lactogène placentaire ont été réalisés en une seuel étape avec le glutaraldéhyde selon le protocole suivant:1 mg d'HLP et 1 mg de $^{\Delta}$5-3-céto stéroïde isomérase, en solution dans 1 ml de tampon phosphate pH 7,2 sont incubés avec 2% ou 0,02% de glutaraldéhyde à température ambiante pendant 24 heures. Après réaction, les conjugués sont séparés par chromatographie d'affinité sur Sepharose oestradiol puis sur Sephadex G 100.

Les essais de couplage avec le phényldiisocyanate et avec le diméthylsuberimidate ont été réalisés après activation de la $^{\Delta}$5-3-cétostéroïde isomérase par le réactif bifonctionnel (le réactif bifonctionnel à la concentration $10^{-2}$ M est incubé pendant 2 heures à température ambiante avec la $^{\Delta}$5-3-cétostéroïde isomérase ($4,10^{-5}$ M). Après réaction, l'excès de réactif est éliminé par chromatographie sur Sepharose oestradiol, l'enzyme modifiée (1 mg) est alors incubée avec 1 mg d'hormone lactogène placentaire. Après une nuit de réaction à température ambiante, l'excès d'hormone est éliminé par chromatographie d'affinité sur oestradiol Sepharose.

Les résultats obtenus sont portés dans le tableau I.

Les résultats montrent que le glutaraldéhyde à forte concentration dénature l'enzyme et fournit un très mauvais rendement de couplage, et que le glutaraldehyde à faible concentration, tout en évitant partiellement la dénaturation de l'enzyme, fournit un rendement de couplage de l'ordre ne dépassant pas 15% environ. Avec le phényldiisocyanate à pH 7, le rendement est inexistant, tandis qu'à pH 9,5 il y a une dénaturation presque totale de l'enzyme. Avec le diméthylsuberimidate, on note une inactivation partielle et le rendement de couplage est de l'ordre de 15%.

Au contraire, le second couplage permet d'obtenir un rendement de couplage élevé tout en conservant la plus grande partie de l'activité enzymatique. On voit donc qu'un tel couplage est bien adapté à la $^{\Delta}$5-3-cétostéroïde isomérase alors que, en raison des caractères particuliers de cette enzyme, les autres réactifs de couplage, bien que largement utilisés dans les couplages enzymatiques, sont moins intéressants du point de vue pratique.

On notera également que le mode de couplage impliquant un pont disulfure, pourrait être utilisé avec d'autres enzymes utilisables comme marqueurs. Au cours des essais effectués on a cependant constaté d'une part que toutes les enzymes ne conviennent pas et, d'autre part, que certaines des enzymes qui conviennent donnent lieu ultérieurement à des difficultés dans la purification des produits conjugués obtenus du fait que ces enzymes ne peuvent être purifiées par chromatographie d'affinité. Ainsi, des essais ont été conduits avec l'hormone lactogène placentaire mais avec diverses enzymes, à savoir la peroxydase, la phosphatase et la stéroïde déshydrogénase à titre d'enzymes connues de comparaison, et la $^{\Delta}$5-3-céto stéroïde isomérase.

Le couplage de la peroxydase, de la phosphatase et de la stéroïde déshydrogénase est réalisé après thiolation de ces enzymes par l'anhydride S-acétyl-mercaptosuccinique (voir exemple 2 ci-après). Les résidus thiols sont activés sous forme de disulfures mixtes avec le dithiobisnitrobenzoate. L'enzyme

activée est séparée du dithiobisnitrobenzoate en excès par chromatographie sur Sephadex G 25; la fraction contenant l'enzyme activée est incubée avec 1 mg d'HLP préalablement traitée au mercaptobutyrimidate (voir exemple 2). La réaction est suivie par spectrométrie à 412 nm et les produits de couplage sont séparés par chromatographie sur Sephadex G100.

Les résultats obtenus sont rassemblés dans le tableau II.

Les résultats du tableau II montrent qu'avec le mode de couplage proposé, la peroxydase et la phosphatase seraient en principe utilisables comme marqueurs pour les dosages immunoenzymatiques, mais l'immunoréactivité du conjugué obtenu après purification n'est pas satisfaisante. Quant à la déshydrogénase, elle ne peut être utilisée avec ce mode de couplage, du fait de son inhibition par le réactif de couplage. Il en serait de même des enzymes possédant un résidu de cystéine essentiel à l'activité.

On voit donc que le mode de couplage préféré tient compte des caractères particuliers de la $^{\Delta}5$-3-cétostéroïde isomérase. En effet, le choix combiné de celle-ci et du mode de couplage permet d'obtenir simultanément deux avantages essentials:

— rendement élevé dans l'obtention des conjugués

— purification facile, par chromatographie d'affinité, permettant d'obtenir des conjugués possédant une très forte immunoréactivité.

On a indiqué précédemment que, pour le dosage immunoenzymatique proprement dit, il était possible d'utiliser les techniques habituelles, par exemple la technique dite du double anticorps. Ainsi, on peut faire appel au protocole déjà employé pour le dosage radio-immunologique, l'hormone radioactive ayant été remplacée par le conjugué $\Delta_5$-3-céto stéroïde isomérase.

Selon un mode de réalisation faisant appel à des moyens conventionnels, en vue du dosage, après séparation et lavage par centrifugation, l'antigène lié à l'immunoabsorbant est incubé. Après centrifugation, l'activité enzymatique du surnageant est déterminée, ce qui peut se réaliser automatiquement à l'aide d'un spectophotomètre. Les variations de l'absorbance à 248 nm en fonction de la quantité d'hormone ou autre substance à tester permettent d'effectuer un dosage quantitatif.

Il faut noter que l'activité enzymatique correspondant à la fixation non spécifique est remarquablement faible du fait que seule l'enzyme retenue spécifiquement par l'immunoabsorbant peut être libérée, par exemple par le dithiotreitol, alors que l'enzyme retenue de façon non spécifique reste liée à la phase. Cet avantage permet d'utiliser la préparation enzyme-antigène directement obtenue après chromatographie d'affinité et évite la chromatographie sur Sephadex G100 qui sépare l'enzyme libre de l'enzyme conjuguée.

TABLEAU I

Couplage avec la $\Delta_5$-3 cétostéroïde isomérase

| | Glutaraldéhyde 2% | Glutaraldéhyde 0,02% | Phényldiisocyanate pH = 7 | Phényldiisocyanate pH = 9,5 | Diméthyl-suberimidate | Invention |
|---|---|---|---|---|---|---|
| Activité résiduelle | 10% | 40% | 60% | <5% | 40% | 80 à 70% |
| % HLP couplé à l'isomérase | <1% | 15% | <0,5% | non déterminé | 15% | 60% |

TABLEAU II

Couplage de l'hormone lactogène placentaire avec différentes enzymes

| | $\Delta_5$-3-céto stéroïd isomérase | Peroxydase | Phosphatase | Stéroïd déshydrogénase |
|---|---|---|---|---|
| Activité résiduelle | 70% | 80% | 50% | 30% |
| Immunoréactivité du conjugué obtenu après purification | 90% | 30% | 40% | non déterminé |

Le résultat obtenu montre également qu'il existe une bonne corrélation entre le dosage immunoenzymatique selon l'invention et la méthode radioimmunologique. En effet, la précision de la mesure, dans le cas par exemple de l'hormone lactogène placentaire, est au moins égale à celle fournie par un dosage radioimmunologique.

Cette constatation a été vérifiée quelle que soit la méthode de séparation utilisée, par exemple la méthode des anticorps insolubilisés ou la méthode par double anticorps.

Il faut noter cependant que l'invention permet aussi de réaliser des dosages immunoenzymatiques dans des conditions qu'il n'était pas possible jusqu'à présent de mettre en oeuvre, précisément en raison de la nature des enzymes utilisées. Dans toutes les méthodes connues, en effet, il était usuel d'utiliser une phase solide, l'enzyme étant fixée sur ce support, et ensuite d'effectuer une incubation, par exemple pendant au moins une dizaine de minutes. Dans bien des cas, cette technique ne permet pas une automatisation complète du dosage. Au contraire, les propriétés particulières de la $^{\Delta}$5-3-céto stéroïde, isomérase, telle qu'elle est appliquée selon l'invention pour les dosages immunoenzymatiques, permettent en général d'éviter cette étape d'incubation. L'invention propose alors un nouveau procédé de dosageedans lequel il suffit de faire passer la solution de substrat sur une phase solide contenant l'enzyme insolubilisée. Par exemple, ce passage peut impliquer une simple filtration ou percolation sur un lit de support tel que le "Sepharose", contenant l'enzyme insolubilisée. Il suffit alors de mesurer l'absorbance de la solution du substrat après son passage sur cette phase solide. L'homme de l'art comprendra qu'un tel procédé de dosage se prête parfaitement à une automatisation complète, en particulier sur des appareils déjà connus et utilisés en radioimmunologie, par exemple l'appareil mis sur le marché sous la dénomination "Centria" par la Société UNION CARBIDE.

On a indiqué précédemment que l'utilisation de la $^{\Delta}$5-3-cétostéroïde isomérase permet un nouveau procédé de dosage immunoenzymatique, à savoir un procédé ne faisant pas appel à des anticorps insolubilisés.

Le complexe antigène-enzyme-anticorps peut être séparé de l'enzyme-antigène par précipitation différentielle par des solvants tels que: dioxane, éthanol, méthanol, ou plus particulièrement polyéthylène-glycol. Le conjugué antigène-enzyme reste en solution et peut être dosé directement. Cette technique est applicable au dosage d'antigènes de faible poids moléculaire ($\leqslant$ 25000).

Le mode de couplage rend possible un tel procédé de dosage, car il permet d'obtenir un conjugué antigèneenzyme possédant une immunoréactivité supérieure à 50%, ou, en d'autres termes, il assure qu'un taux d'activité enzymatique supérieur à 50% peut être lié aux anticorps.

Ainsi, grâce à l'application de la $^{\Delta}$5-3-cétostéroïde isomérase qui est proposée par l'invention pour les dosages immunoenzymatiques, on peut non seulement réaliser des dosages traditionnels, mais il devient également possible de mettre en oeuvre de nouvelles techniques de dosage. C'est ainsi que la révélation de l'activité enzymatique liée aux anticorps peut être réalisée par plusieurs moyens:

1) lecture directe sur le surnageant ou sur le culot obtenu par précipitation à l'aide d'un solvant, tel que le polyéthylène glycol, avec l'utilisation d'anticorps solubles;

2) lecture en solution après libération de l'enzyme du complexe antigène-anticorps par un réducteur tel que le dithiotréitol;

3) lecture immédiate après filtration d'une solution de substrate sur le complexe insolubilisé anticorps-antigène-enzyme.

Les indications précédentes feront suffisamment apparaître à l'homme de l'art l'intérêt de ces nouvelles techniques de dosage et la manière de les utiliser.

Quelle que soit la méthode de dosage utilisée, la haute activité spécifique de l'enzyme permet de mettre en oeuvre des quantités du complexe antigène-enzyme qui se trouvent à l'état de traces dans le milieu réactionnel. En fait, l'immunoréactivité du complexe est comparable à celle de l'antigène libre du fait du faible poids moléculaire de l'enzyme et de la sélectivité du couplage.

L'utilisation pour le dosage immunoenzymatique de la $\Delta_5$,3-céto-stéroïde isomérase présente de nombreux avantages dont les principaux peuvent être résumés comme suit:

— enzyme d'activité spécifique très élevée allant jusqu' à 60.000 UI/mg, ce qui correspond à une variation de densité optique à 248 nm, par minute, de 24.000 milliunités d'absorbance pour une picomole;

— faible perte d'activité enzymatique lors du couplage;

— purification et séparation du complexe Ag-Enz de Ag en excès par une chromatographie d'affinité simple et rapide;

— stabilité du complexe Ag-Enzyme

— 0,5 mg de complexe Ag-Enzyme permettent de réaliser près de $10^6$ dosages;

— faible poids moléculaire ne diminuant pas l'immunoréactivité de l'antigène;

— la révélation de l'activité enzymatique étant réalisée en quelques minutes, le dosage est automatisable rapidement sur les appareils existant pour la radioimmunologie.

L'invention sera maintenant illustrée sans être aucunement limitée par les exemples qui suivent:

Exemple 1

Préparation de la $\Delta_5$,3-céto stéroïde isomérase

*1a. Culture du microorganisme*

On a cultivé le microorganisme *Pseudomonas Testosteroni* (A.T.C.C.11996) à 30°C dans un milieu liquide de composition suivante:extrait de levure Difco, 10 g; $(NH_3)_2HPO_4$, 1 g; $(NH_3)H_2PO_4$, 1 g; $KH_2PO_4$, 2 g; et 10 ml d'une solution d'éléments minéraux par litre d'eau désionisée. La composition de la solution minérale était: $MgSO_4 . 7 H_2O$, 20 g; NaCl, 1 g; $ZnSO_4 . 7 H_2O$, 0,5 g; $MnSO_4 . 3 H_2O$, 0,5 g; $CuSO_4 . 5 H_2O$, 0,05 g; 0,1 N $H_2SO_4$, 10 ml dans un litre d'eau distillée. Le pH final a été ajusté à 6,65 avec NaOH 1 N, et le miliéu stérilisé à 121°C pendant 30 minutes. La culture a été réalisée dans des cuves d'une capacité de 500 litres, contenant chacune 250 litres de milieu. On a ajouté d'emblée 0,2 ml par litre de culture d'un agent antimousse ("Antifoam A—60" — General Electric Company), et ultérieurement des quantités variables selon nécessité. Les cuves ont été aérées à raison de 125 à 250 litres par minute et agitées à raison de 260 rotations par minute (rpm). Chaque cuve a été ensemencée avec 2,5 litres (1% en volume) d'une culture vieille de 18 heures du microorganisme cultivé sur le même milieu.

Après 7 heures et demie de culture, 75 g de progestérone finement pulvérisée et suspendue dans environ 600 ml de "Tween 60" à 1% ont été introduits dans chaque réservoir (300 mg par litre). La culture a alors été poursuivie pendant une durée totale de 23 heures à partir du moment de l'inoculation.

On a refroidi ensuite le contenue des cuves, par une circulation d'eau, jusqu' à 8 à 10°C. Les cellules ont été recueillies par centrifugation à 13.500 r.p.m. dans une supercentrifugeuse "Sharples" à débit continu, ayant un réservoir d'une capacité de 6 litres. Le débit était de 2,4 à 2,6 litres par minute, et a permis la sédimentation de 93 à 94% des cellules. Le produit de chaque cuve a rempli le rotor au tiers ou à la moitié de sa capacité. Le sédiment cellulaire a finalement été transféré du rotor dans des bocaux en matière plastique d'une contenance de 500 ml et congelé dans la glace carbonique. Le rendement en substance cellulaire humide a été de 1,9 à 2,2 kg par 250 litres de milieu de culture.

*1b. Préparation de la poudre acétonique*

La masse cellulaire obtenue conformément à l'exemple 1*a* a été dégelée à 4°C pendant environ 12 heures et suspendue dans 1,5 litre de solution tampon de phosphate de potassium à pH 7,0 pour chaque kilogramme de sédiment, par traitement dans un mélangeur "Waring Blendor" (capacité 4 litres) tournant à vitesse réduite et pendant un temps suffisant pour obtenir une suspension de consistance uniformément crémeuse.

Des quantités aliquotes de cette suspension (250 ml) ont été alors versées, à très faible débit, dans 2,5 litres d'acétone à —20°C placés dans le bol d'un "Waring Blendor", tandis que l'agitation était continuée à petite vitesse.

Après avoir réuni les suspensions, on les a laissées à —20°C pendant une nuit, de manière à permettre une sédimentation des particules. On a éliminé alors par aspiration la plus grande partie du surnageant. La suspension très dense qui restait a été diluée par un volume égal d'acétone à —20°C et versée dans de larges entonnoirs Büchner d'acier inoxydable de 33 cm de diamètre, fournis par "Commercial Equipment Company", Milwaukee, qui avaient été garnis de deux feuilles de papier filtre. On a lavé le résidu avec une quantité supplémentaire d'acétone à —20°C puis avec de l'éther également à —20°C.

La galette obtenue a alors été retirée du Büchner et séchée dans une hotte, par pressage sur une épaisse feuille de papier filtre, de manière à la transformer en une poudre légère, sèche, et qui demeure faiblement colorée en beige.

Cette poudre a été placée dans de larges boîtes de Petri qu'on a maintenues sous vide, dans un dessicateur, jusqu'à ce que toute odeur d'acétone ait disparu. La poudre a été finalement conservée dans une cloche à vide à —20°C. Dans ces conditions, l'activité enzymatique est stable pendant au moins un an.

Le rendement en poudre acétonique a été d'environ 25% du poids total de substance cellulaire obtenue par centrifugation.

*1c. Purification de l'isomérase à partir de la poudre acétonique*

On a procédé ensuite à la purification de l'isomérase en partant de la poudre acétonique préparée conformément à l'exemple 1b. La figure 1 annexée est un tableau qui résume les principales étapes de la purification, celles-ci ayant été opérées sur 100 g de poudre acétonique. L'homme de l'art pourra se reporter à cette figure pour obtenir toutes les informations qui lui sont nécessaires pour l'obtention d'une $\Delta_5$,3-cétostéroïde isomérase de haute pureté.

Le tableau III qui suit résume les résultats obtenus après chacune des étapes de la figure 1.

L'exemple 1 montre que pour 250 l de milieu de culture, on a recueilli 1,9 à 2,2 kg de substance cellulaire humide. Après traitement de cette masse pour obtenir la poudre acétonique, le rendement est de 250 g de poudre/kg de substance cellulaire. A partir de 100 g de poudre acétonique, on peut obtenir 8 mg d'isomérase ayant une activité spécifique de 30.000 UI/mg. On obtient ainsi l'isomérase

appliquée selon l'invention avec une haute activité spécifique et un faible poids moléculaire (25.000 environ).

Exemple 2

Couplage de la $\Delta_5$,3-cétostéroïde isomérase avec une hormone peptidique, l'hormone lactogène placentaire HLP.

TABLEAU III

Purification de l'isomérase (essai sur 100 g de poudre)

| | Volume | Unités ACT/ML | Activité totale | Absorbance | Activité spécifique UI/MG | Rendement total | Purification |
|---|---|---|---|---|---|---|---|
| Extraction | 650 | 1100 | $715.10^3$ | 52,3 | 21 | | |
| après précipitation (70% $SO_4(NH_4)_2$) | 370 | 1840 | $683.10^3$ | 41,6 | 44 | 95,5 | 2,1 |
| après dialyse | 450 | 1290 | $580.10^3$ | 21,4 | 63,23 | 81,1 | 3,01 |
| après DEAE | 310 | 1860 | $575.10^3$ | 11 | 169 | 80,4 | 8,04 |
| après chromatographie d'affinité | 80 | 2900 | $232.10^3$ | 0,05 | 30 000 | 30,8 | 1500 |

*2a. Fixation des résidus thiolssur l'HLP*

Dans cet exemple, on fixe comme suite les résidus thiols sur l'HLP par amidination avec le 4-mercaptobutyrimidate de méthyle: 2 mg d'HLP sont dissous dans 300 $\mu$l d'eau amenée à pH 9,5 avec 100 $\mu$l de tampon carbonate-bicarbonate 0,1 M et additionnés de 1,3 mg de 4-mercaptobutyrimidate de méthyle. Après 4 heures de réaction, la solution est dialysée 2 fois contre 250 ml de tampon phosphate 0,03 M, pH 7,2, dithiotreitol (DTT) $10^{-6}$ M. Après dialyse, 50 $\mu$l de la solution réactionnelle sont prélevés et les groupements thiols sont dosés par le réactifs d'ELLMAN.

*2b. Fixation de résidus dithionitrobenzoate sur l'enzyme*

Les résidus dithionitrobenzoate sont introduits sur l'enzyme comme suit:1,6 mg d'isomérase (activité totale:50000 UI) contenue dans 2 ml de tampon pH 7,2 0,1 M, 20% glycérol sont incubés avec 3,5 mg d'anhydride S-acétyl-mercapto succinique (SAMSA) pendant 2 heures à température ambiante. Puis, la solution est dialysée 2 heures contre 200 ml de tampon phosphate 0,03 M pH 7,2 puis 2 heures contre 100 ml d'hydroxylamine 0,1 M pH 7,4 $10^{-2}$ M DTT et enfin une nuit contre 1 litre de tris HCl 0,1 M pH 8 $10^{-6}$ M DTT. La solution d'isomérase est incubée avec $10^{-3}$ M 5,5'-dithio-bis (2 nitro benzoate), l'absorbance à 412 nm est déterminée à l'aide d'un spectrophotomètre Perkin-Elmer.

*2c. Couplage HLP-Enzyme*

L'enzyme modifiée est séparée de l'excès de réactif par chromatographie d'affinité sur une colonne de 0,5 ml de Sepharose oestradiol 17 $\beta$. L'excès de réactif d'ELLMAN est éliminé par lavage par 30 ml de tampon 0,1 M phosphate, pH 7,2. L'enzyme est éluée par 2 ml d'eau désionisée contenant 20% de glycérol. L'enzyme est recueillie dans 1,4 ml qui sont ajoutés directement à la solution d'HLP, précédemment obtenue (0,5 ml), le pH de la solution étant amené à 8,6 pour favoriser l'échange des ponts disulfures. La réaction est suivie par la mesure de l'absorbance à 412 nm due à la libération de thionitrobenzoate. Après une nuit d'incubation, l'excès d'HLP est séparé de la fraction liée à l'enzyme par une nouvelle chromatographie d'affinité sur oestradiol Sépharose; l'excès d'hormone est éliminé par lavage par 50 ml de tampon phosphate 0,1 M. L'enzyme libre et l'enzyme conjuguée à l'hormone sont éluées par 2 ml d'eau contenant 20% de glycérol. L'enzyme libre peut être séparée du conjugué enzyme-hormone par chromatographie sur Sephadex G 100.

Exemple 3

Dosage immunoenzymatique de l'HLP

Dans cet exemple on a utilisé soit les anticorps anti HLP insolubilisés (mis sur le marché par BIOMERIEUX) soit la méthode du double anticorps (UNION CARBIDE). Le protocole qui a été suivi est le même que celui proposé par le fabricant pour le dosage radioimmunologique. Toutefois, l'hormone radioactive a été remplacée par le conjugué hormone isomérase préparé à l'exemple 2c. Ce conjugué a été réparti à raison de 0,1 UI/tube.

Après séparation et lavage par centrifugation, l'antigène lié à l'immunoabsorbant est incubé avec 100 $\mu$l de tampon phosphate $10^{-2}$ M DTT pendant environ 1 heure puis additionné de 0,9 ml de tampon phosphate 0,03 M contenant 100 mg par litre d'un produit détergent mis sur le marché par ICI (USA) sous la dénomination Brij 35.

Après centrifugation, l'activité enzymatique du surnageant est déterminée automatiquement de la manière suivante à l'aide d'un spectrophotomètre Perkin-Elmer couplé à unéchantillonneur et à un distributeur automatique Gilson.

—500 $\mu$l de tampon phosphate 0,03 M, pH 7,2 contenant 100 mg/litre de Brij 35,200 ml/litre d'éthanol et $4.10^{-4}$ M de $\Delta_5$ androstène-dione sont ajoutés automatiquement à 1 ml du surnageant contenant l'enzyme. L'activité enzymatique est déterminée par mesure de l'absorbance à 248 nm après 10 minutes d'incubation.

Les résultats obtenus sont illustrés aux figures 2 à 4.

La figure 2 est un graphique illustrant la variation d'absorbance à 248 nm en fonction de la quantité d'hormone exprimée en nanogrammes La courbe obtenue à la figure 2 résulte du dosage de l'HLP avec une technique connue de dosage de double anticorps.

La figure 3 est un diagramme sur lequel on a porté en abscisses la quantité d'hormone lactogène placentaire et en ordonnées d'une part l'absorbance à 248 nm selon les résultats fournis par le dosage immunoenzymatique de l'invention (courbe 1 référence EIA) et par ailleurs les résultats obtenus par une méthode radioimmunologique où l'on a évalué la radioactivité (en coups par minute cpm) ce qui a fourni la courbe 2 référence RIA.

La figure 4 est un graphique destiné à montrer la bonne corrélation des résultats obtenus par le dosage immunoenzymatique de l'invention et ceux de la méthode radioimmunologique. On a porté sur ce graphique, en abscisse, les quantités déterminées par la méthode radioimmunologique, et en ordonnée les quantités d'hormones HLP déterminées par le dosage immunoenzymatique.

Exemple 4

Dosage de la progestérone

*4a. Synthèse du conjugué progestérone $\Delta_5$,3-céto stéroïde isomérase*

On commence par préparer d'une manière connue le 11$\alpha$-bromoacétate de progestérone par la technique décrite dans C.R. Acad. Sc. Paris t.283 (8 Novembre 1976) Série C,p. 507—510. D'une manière résumée on traite la 11$\alpha$ OH-progestérone par l'acide bromoacétique en utilisant le dicyclohexyl-carbodiimide comme agent couplant et le tétrahydrofuranne comme solvant.

Par ailleurs, on utilise une partie du mode opératoire décrit à l'exemple 2b pour fixer des groupements-SH sur l'enzyme.

A cet effet, on utilise 1 mg de $\Delta_5$,3-céto-stéroïde isomérase. On incube l'isomérase contenue dans 2 ml de tampon pH 7,2 0,1 M 20% glycérol avec 3,5 mg d'anhydride S-acétyl-mercapto succinique (SAMSA)pendant 2 heures à température ambiante. Puis, la solution est dialysée 2 heures contre 200 ml de tampon phosphate 0,03 M pH 7,2 puis 2 heures contre 100 ml d'hydroxylamine 0,1 M pH 7,4 $10^{-2}$ M DTT et enfin une nuit contre 1 litre de tris HCl 0,1 M pH 8 $10^{-6}$ M DTT.

En vue du couplage, on incube l'isomérase porteuse de groupements-SH dans 1 ml de tampon phosphate 0,03 M, pH 8 avec $10^{-4}$ M de 11$\alpha$-bromoacétate de progestérone pendant 16 heures à température ambiante. L'excès de stéroïde est éliminé par dialyses répétées (3 dialyses contre 1 litre de tampon phosphate pH 7,2 0,03 M).

*4b. Dosage de la progestérone*

En vue du dosage proprement dit, on peut utiliser deux méthodes, soit la méthode du double anticorps insolubilisé, soit la précipitation spécifique de la fraction $\gamma$-globulines par le polyéthylène glycol.

Les anticorps antiprogestérone utilisés (obtenus contre un conjugué 11 hémisuccinate BSA) sont ceux commercialisés par la firme BIOMERIEUX.

Au lieu d'utiliser une hormone radioactive, comme dans les dosages radioimmunologiques, le conjugué hormone isomérase est mis en oeuvre à raison de 0,05 UI/tube, selon l'une ou l'autre méthode.

*(1) Méthode du double anticorps insolubilisé*

Après incubation et lavage de l'immunoadsorbant, 1'activité enzymatique liée à l'immunoadsorbant est mesurée par incubation pendant 5 minutes de la phase avec le tampon contenant le substrat. Après centrifugation, l'absorbance du surnageant est mesurée à 248 nm.

*(2) Précipitation par le polyéthylène-glycol*

Après incubation de l'anticorps avec le conjugué enzyme-progestérone, obtenu à l'exemple 4b, et l'échantillon à doser, la fraction $\gamma$-globulines est précipitée par addition d'une solution de polyéthylène-glycol (concentration finale 12,5% en poids).

On refroidit 45 minutes à 4°C, puis les tubes sont centrifugés et l'activité enzymatique est mesurée dans le surnageant.

La figure 5 est un graphique où l'on a porté les résultats d'un dosage représentatif de la progestérone par la méthode 4b(1). L'absorbance à 248 nm a été portée en ordonnées et la concentration en progestérone, exprimée en picogrammes, se trouve en abscisses.

Exemple 5

A l'aide d'un appareillage particulier mis sur le marché par la Société Union Carbide, dénommé "Centria", on a procédé à une comparaison directe entre la méthode de dosage radioimmunologique, l'hormone ayant été marquée par l'iode et la méthode de dosage immunoenzymatique réalisée selon l'invention, l'hormone ayant été marquée par la $\Delta_5$,3-céto stéroïde isomérase.

Les conditions utilisées pour ce dosage sont celles préconisées par le fabricant pour le dosage radioimmunologique. L'antigène conjugué à l'isomérase, l'antigène et les anticorps spécifiques sont incubés pendant 1 heure. Les anticorps sont ensuite retenus spécifiquement par un immunoadsorbant (colonne de Sépharose anticorps anti- $\gamma$ globulines de lapin). L'immunoadsorbant est lavé par du tampon contenant 1% de détergent (Tween 80). L'activité enzymatique liée à l'immunoadsorbant est déterminée par passage d'une solution $10^{-4}$ M de $\Delta_5$ androstènedione à raison de 2 ml en 5 minutes. L'absorbance à 248 nm du filtrat est déterminée à l'aide d'un spectrophotomètre Perkin Elmer.

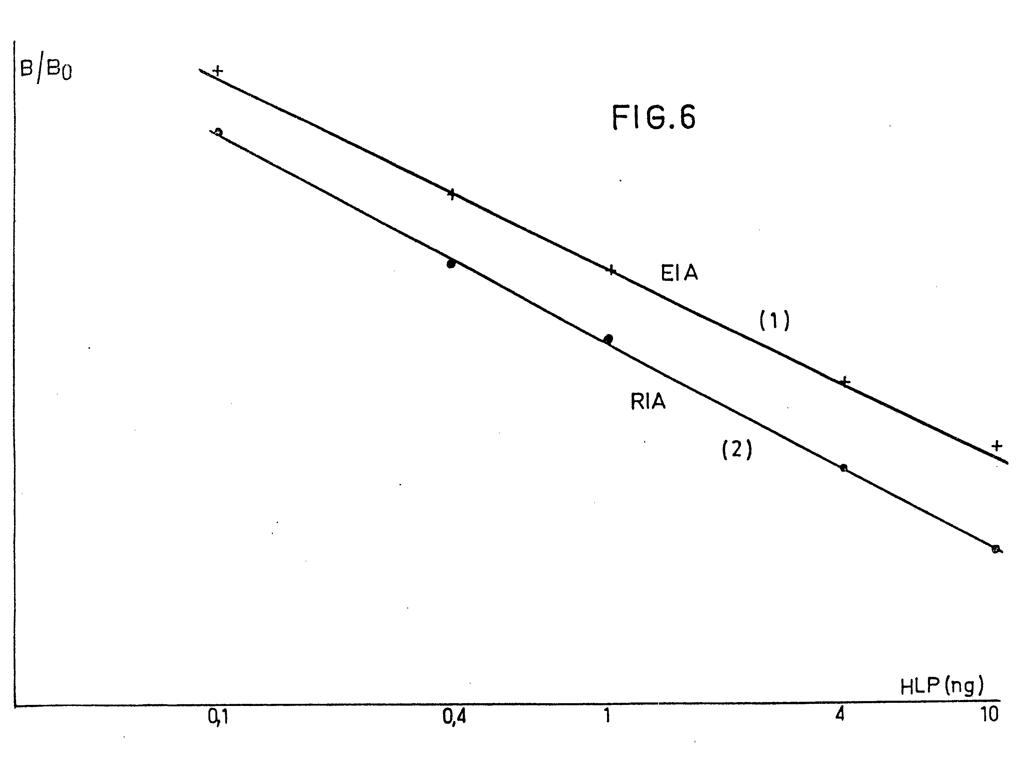

La figure 6 illustre les résultats obtenus avec l'hormone lactogène placentaire. Cet exemple d'application illustre la faculté qu'offre le dosage selon l'invention d'être exploité par voie entièrement automatique. On a réalisé une gamme de concentrations allant de 10 ng à 0,1 ng. Dans cette gamme, les deux marqueurs ont été utilisés simultanément. Les courbes obtenues sont indiquées respectivement courbe 1 pour le dosage immunoenzymatique selon l'invention et courbe 2 pour le dosage radioimmunologique. Les résultats montrent que l'antigène marqué par l'isomérase présente peu d'adsorption non spécifique et possède une immunoréactivité comparable à celle de l'hormone radioactive.

Dans le graphique de la figure 6, on a porté en abscisses la quantité de HLP exprimée en ng et en

ordonnées le rapport B/Bo de l'activité enzymatique ou radioactive de la fraction liée à la même activité de la fraction liée au maximum, c'est-à-dire en l'absence de tout antigène.

**Revendications**

1. Application de la $\Delta_5$,3-céto-stéroïde isomérase comme marqueur enzymatique dans les dosages immunoenzymatiques, l'activité enzymatique, qui constitue une valeur représentative de la concentration de la substance à mesurer, étant déterminée par une mesure d'absorption dans l'ultraviolet à 248 nm.

2. Application selon la revendication 1 au dosage immunoenzymatique de substances présentes à très faible concentration dans des milieux biologiques, telles que les hormones hypophysaires et les stéroïdes plasmatiques.

3. Application selon l'une des revendications 1 ou 2 au cas où l'antigène à doser est une substance de nature peptidique, telle qu'une hormone peptidique, par exemple l'hormone placentaire humaine HLP, ladite application étant caractérisée en ce qu'on réalise un couplage par pont disulfure en faisant réagir d'une part la substance de nature peptidique et le 4-mercapto-butyrimidate de méthyle, et d'autre part la $\Delta_5$,3-cétostéroïde isomérase et et l'anhydride S-acétyl-mercaptosuccinique, et en couplant les produits respectifs de réaction, ce qui conduit à un conjugué dans lequel une mole d'enzyme est couplée à une mole de substance peptidique et qui conserve au moins 50% de son activité enzymatique après purification, celleci étant effectuée de manière connue par chromatographie d'affinité.

4. Application selon l'une des revendications 1 ou 2 au cas où l'antigène à doser est une substance de nature stéroidique, telle qu'une hormone stéroïde, par exemple la progestérone, ladite application étant caractérisée en ce qu'on réalise un couplage par l'intermédiaire d'un résidu thio en faisant réagir d'une part la substance de nature stéroïdique avec l'acide bromoacétique de formule $Br—CH_2—COOH$, en présence de carbodiimide, et d'autre part la $\Delta_5$,3-cétostéroïde isomérase avec l'anhydride S-acétyl-mercaptosuccinique et en couplant les produits respectifs de réaction.

5. Application selon la revendication 3, caractérisée en ce que l'enzyme est libérée du complexe antigène-enzyme-anticorps sous l'action d'un réducteur, tel que le dithiotreitol ou le $\beta$-mercapto-éthanol, et en ce qu'on mesure l'activité enzymatique en phase homogène.

6. Application selon la revendication 3, caractérisée en ce qu'on sépare le complexe antigène-enzyme-anticorps du conjugué enzyme-antigène par précipitation différentielle à l'aide d'un solvant, tel que le dioxane, le méthanol, l'éthanol ou, de préférence, le polyéthylène glycol, et en ce qu'on mesure directement l'activité enzymatique sur le surnageant ou sur le culot de précipitation.

7. Application selon la revendication 6 en vue du dosage d'antigènes de poids moléculaire inférieur ou égal à 25.000 environ.

8. Application selon la revendication 3, caractérisée en ce qu'on utilise une phase solide sur laquelle est fixée la $\Delta_5$,3-cétostéroïde isomérase au sein d'un complexe insolubilisé anticorps-enzyme-antigène, en ce qu'on fait directement passer une solution de substrat sur ladite phase solide, par exemple par filtration ou percolation, et en ce qu'on mesure immédiatement l'absorbance à 248 nm de la solution de substrat après son passage sur la phase solide.

9. Application selon l'une quelconque des revendications 1 à 8, caractérisée en ce qu'on effectue le dosage d'une manière automatique.

10. $^{\Delta}5$-3-cétostéroïde-isomérase modifiée, mise en oeuvre comme réactif de couplage dans l'application selon l'une quelconque des revendications 3 à 9, caractérisée en ce qu'elle porte des résidus thiol.

**Patentansprüche**

1. Verwendung von $\Delta_5$,3-Ketosteroidisomerase als enzymatische Markierung in immunoenzymatischen Dosierungen, wobei die enzymatische Aktivität, die einen für die Konzentration der zu messenden Substanz representativen Wert bildet, durch Absorptionsmessung im Ultravioletten bei 248 nm bestimmt wird.

2. Verwendung nach Anspruch 1 bei der immunoenzymatischen Dosierung von Substanzen, die in sehr geringer Konzentration in biologischen Milieus vorhanden sind, etwa Hypophysenhormone und plasmatische Steroide.

3. Verwendung nach Anspruch 1 oder 2 für den Fall, daß das zu dosierende Antigen eine Substanz mit Peptidnatur, etwa ein Peptidhormon, beispielsweise menschliches Placentahormon HLP, ist, wobei die Verwendung dadurch gekennzeichnet ist, daß man eine Kopplung durch Disulfidbrücken vornimmt, indem man einerseits die Substanz mit Peptidnatur und Methyl-4-mercaptobutyrimidat und andererseits $\Delta_5$,3-Ketosteroidisomerase und S-acetylmercaptosuccinsäureanhydrid reagieren läßt und die entsprechenden Reaktionsprodukte koppelt, die zu einer Konjugation führen, in der ein Mol des Enzyms mit einem Mol der peptidartigen Substanz gekoppelt ist und die wenigstens 50% ihrer enzymatischen Aktivität nach Reinigung, die in bekannter Weise durch Affinitätschromatographie vorgenommen wird, beibehält.

4. Verwendung nach Anspruch 1 oder 2 für den Fall, daß das zu dosierende Antigen eine Substanz mit Steroidnatur, etwa ein Steroidhormon, beispielsweise Progesteron, ist, wobei die Verwendung dadurch gekennzeichnet ist, daß man eine Kopplung durch Zwischenschaltung eines Thiorestes vornimmt, indem man einerseits die Substanz mit Steroidnatur mit Bromessigsäure der Formel $Br{\cdot}CH_2{-}COOH$ in Anwesenheit von Carbodiimid und andererseits $\Delta_5$,3-Ketosteroidisomerase mit S-Acetylmercaptosuccinsäureanhydrid reagieren läßt und die entsprechenden Reaktionsprodukte koppelt.

5. Verwendung nach Anspruch 3, dadurch gekennzeichnet, daß das Enzym des Antigen-Enzym-Antikörper-Komplexes unter der Wirkung eines Reduktionsmittels wie Dithiotreitol oder $\beta$-Mercaptoäthanol freigesetzt wird und man die enzymatische Aktivität in homogener Phase mißt.

6. Verwendung nach Anspruch 3, dadurch gekennzeichnet, daß man den Antigen-Enzym-Antikörper-Komplex von der konjugierten Verbindung Enzym-Antigen durch teilweises Niederschlagen mit Hilfe eines Lösungsmittels wie Dioxan, Methanol, Äthanol oder vorzugsweise Polyäthylenglycol trennt und die enzymatische Aktivität direkt an dem Niederschlagsrückstand oder an der abgetrennten Flüssigkeit mißt.

7. Verwendung nach Anspruch 6 zur Dosierung von Antigenen mit Molekulargewichten unterhalb oder gleich etwa 25.000.

8. Verwendung nach Anspruch 3, dadurch gekennzeichnet, daß man eine feste Phase verwendet, auf der $\Delta_5$,3-Ketosteroidisomerase im Rahmen eines unlöslichen Antikörper-Enzym-Antigen-Komplexes befestigt ist, und direkt eine Lösung des Substrats über die feste Phase beispielsweise durch Filtrieren oder Perkolieren führt und unmittelbar die Absoption bei 248 nm der Substratlösung nach dem Passieren der festen Phase mißt.

9. Verwendung nach einem der Ansprüche 1 bis 8, dadurch gekennzeichnet, daß man die Dosierung automatisch vornimmt.

10. Modifizierte $^{\Delta}$5-3-Ketosteroidisomerase, zum Einsatz als Kopplungsreaktionspartner bei den Verwendungen nach einem der Ansprüche 3 bis 9, dadurch gekennzeichnet, daß sie Thiolreste aufweist.

**Claims**

1. Use of $\Delta_5$,3-keto-steroid isomerase as enzymatic labelling agent in immunoenzymatic assays, the enzymatic activity, which constitutes a representative value of the substance to be measured, being determined by the measuring of absorption in the ultraviolet at 248 nm.

2. Use according to claim 1 to the immunoenzymatic assay of substances present in biological media at very low concentration, such as hypophyseal hormones and plasmatic steroids.

3. Use according to one of claims 1 or 2 to the case wherein the antigen to be assayed is a substance of peptide nature, such as a peptide hormone, for example human placental hormone PLH, said use being characterized in that a coupling by a disulphide bridge is effected by reacting on the one hand the substance of peptide nature and methyl 4-mercapto-butyrimidate, and on the other hand $\Delta_5$,3-keto-steroid isomerase and S-acetylmercaptosuccinic anhydride, and by coupling the respective reaction products, which leads to a conjugate in which one mole of enzyme is coupled with 1 mole of peptide substance and which preserves at least 50% of its enzymatic activity after purification, the latter being carried out by conventional means by affinity chromatography.

4. Use according to one of claims 1 or 2, to the case wherein the antigen to be assayed is a substance of steroid nature such as steroid hormone, for example progesterone, said use being characterized in that a coupling is carried out by means of a thio residue by reacting on the one hand the substance of steroid nature with bromoacetic acid of the formula $Br{-}CH_2{-}COOH$, in the presence of carbodiimide, and on the other hand $\Delta_5$,3-keto-steroid isomerase with S-acetylmercaptosuccinic anhydride and by coupling the respective reaction products.

5. Use according to claim 3, wherein the enzyme is released from the antigen-enzyme-antibody complex by the action of a reducing agent, such as dithiotreitol or $\beta$-mercaptoethanol and the enzymatic activity is measured in homogeneous phase.

6. Use according to claim 3, wherein the antigen-enzyme-antibody complex is separated from the enzyme-antigen conjugate by differential precipitation by means of a solvent, such as dioxane, methanol, ethanol or, preferably, polyethylene glycol, and wherein the enzymatic activity is measured directly on the supernatent liquid or on the precipitation cake.

7. Use according to claim 6, for the assay of antigens of molecular weight of about 25,000 or less.

8. Use according to claim 3, wherein a solid phase is used on which the $\Delta_5$,3-keto-steroid isomerase is fixed in the midst of an insolubilized antibody-enzyme-antigen complex, and wherein a substrate solution is passed directly over said solid phase for example by filtration or percolation, and the absorbancy at 248 nm of the substrate solution is immediately measured after its passage over the solid phase.

9. Use according to anyone of claims 1 to 8, wherein the assay is carried out by automatic means.

10. Modified $\Delta_5$,3-keto-steroid isomerase, used as coupling reagent in the use according to any one of claims 3 to 9, wherein its bears thiol residues.

PURIFICATION DE L'ISOMÉRASE SUR 100g DE POUDRE

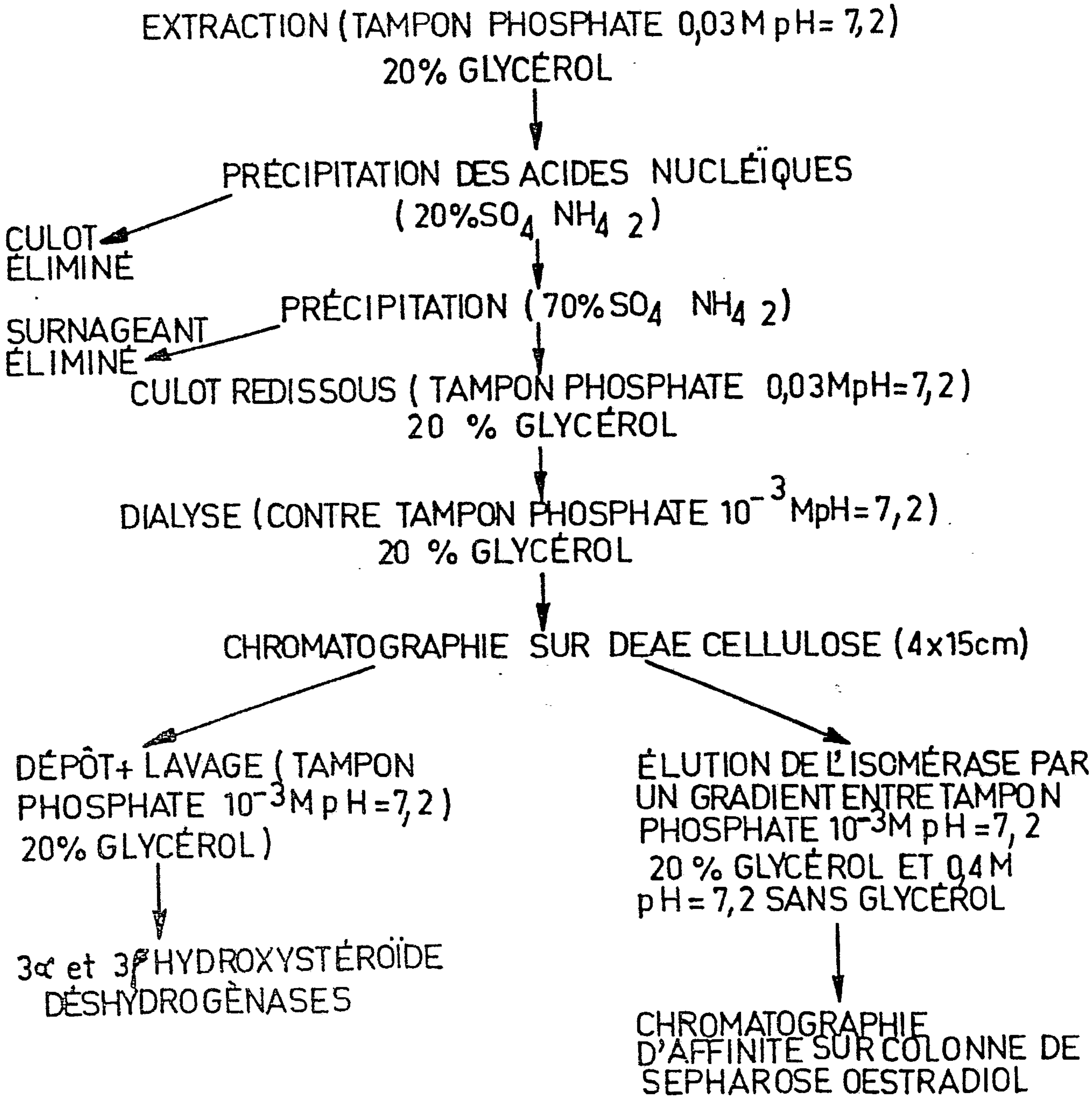


FIG.1

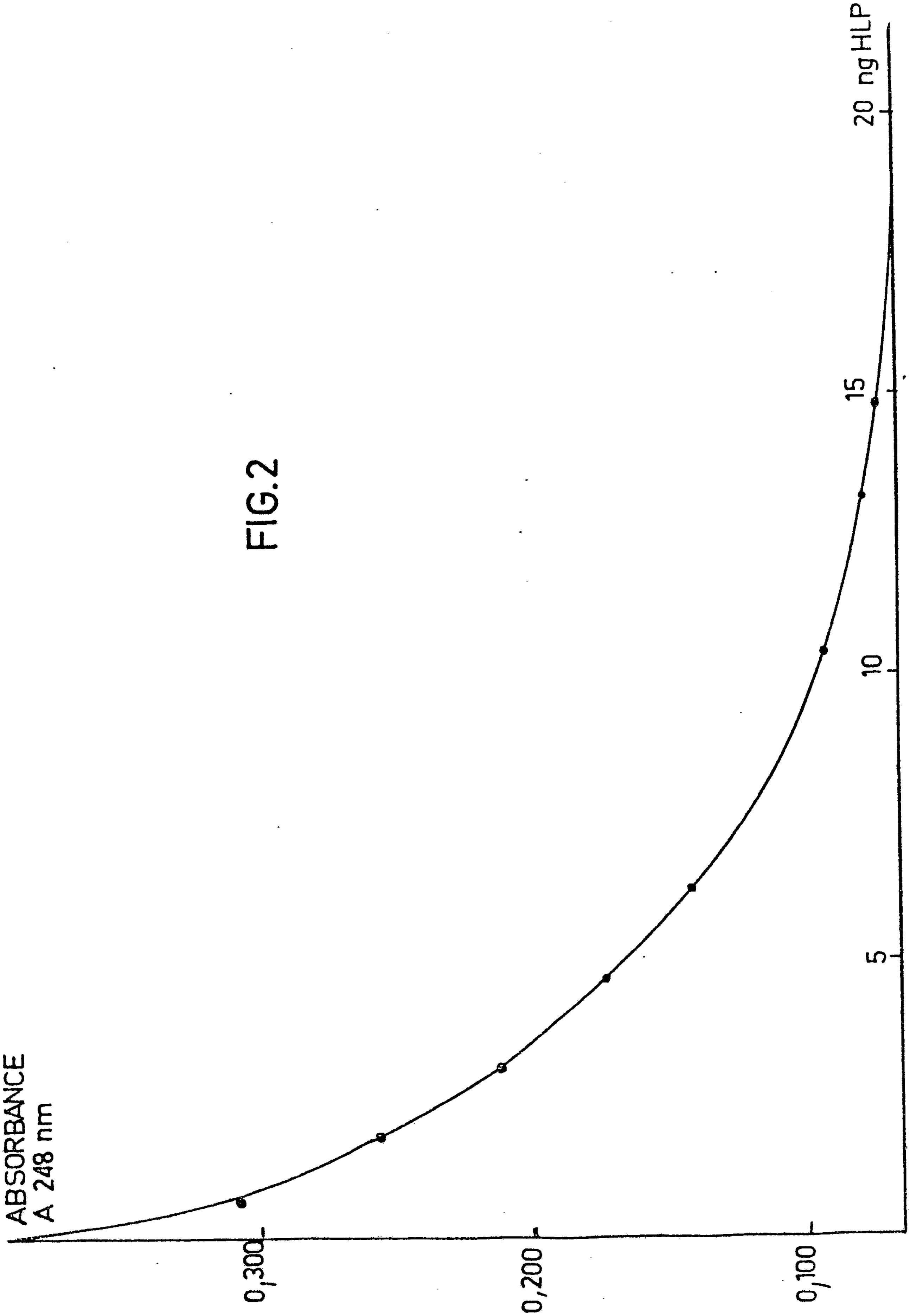


FIG.2

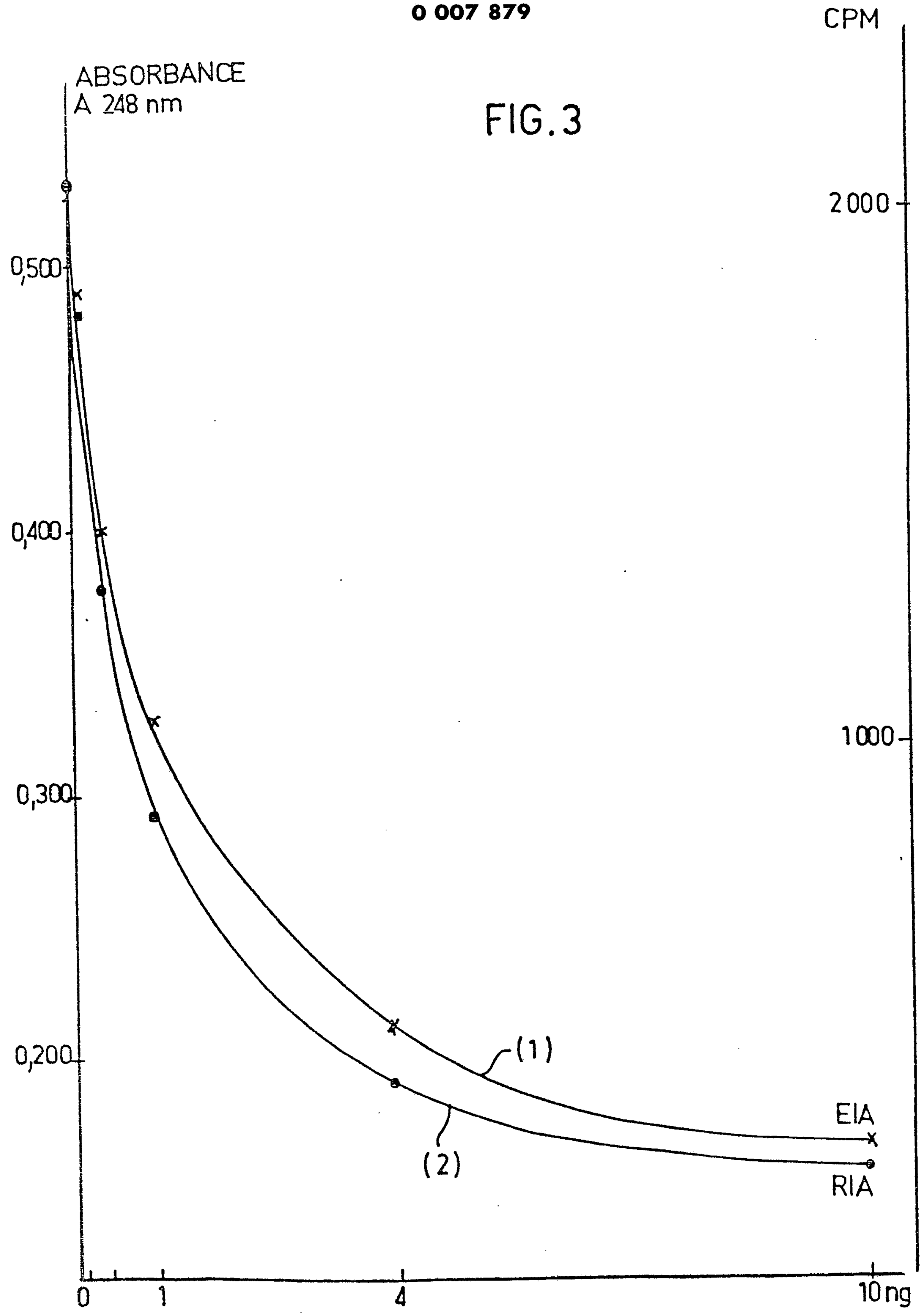
CPM
ABSORBANCE
A 248 nm
2000
0,500
0,400
1000
0,300
0,200
(1)
EIA
(2)
RIA
0
1
4
10 ng

FIG. 3

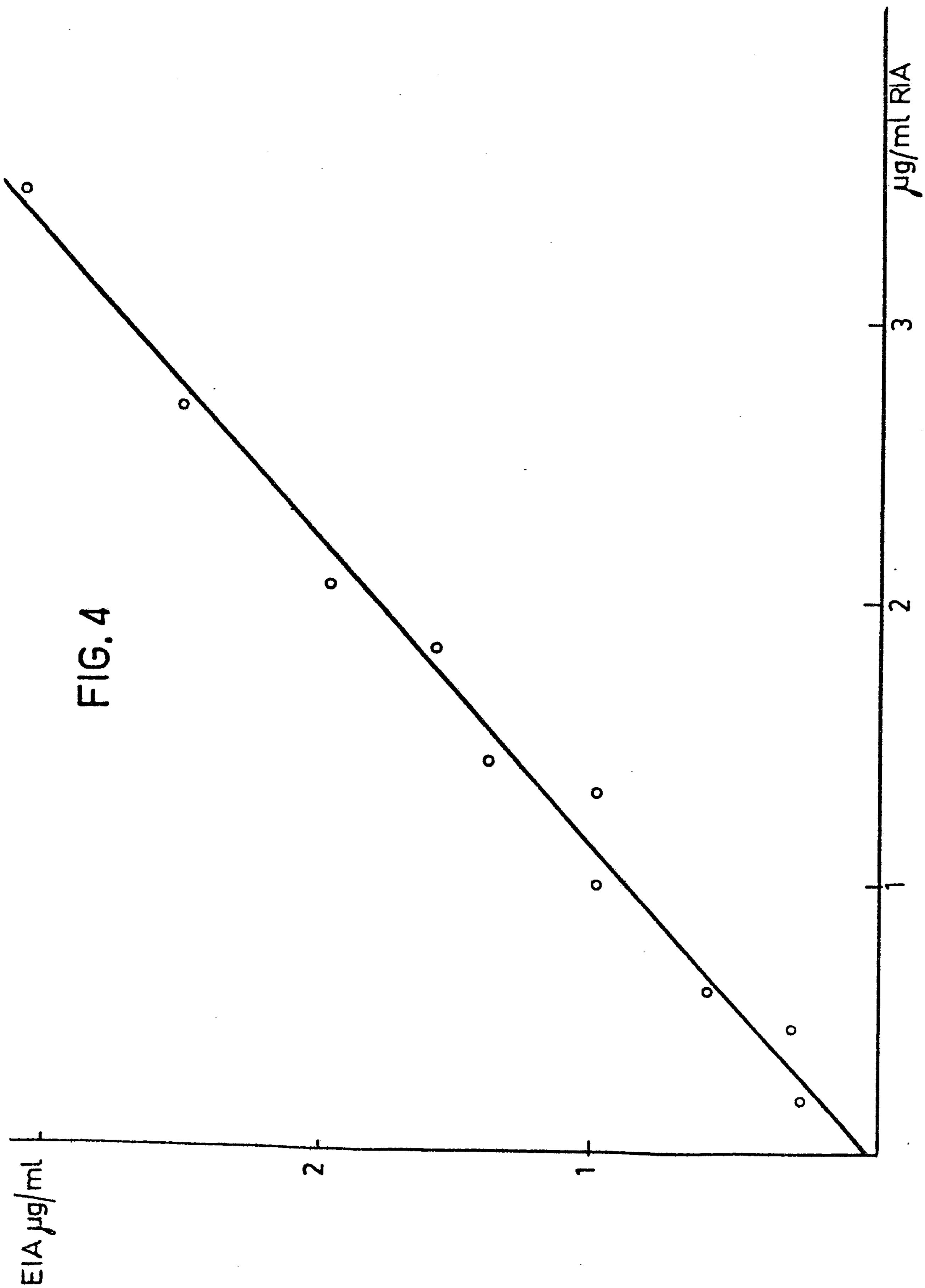


FIG. 4

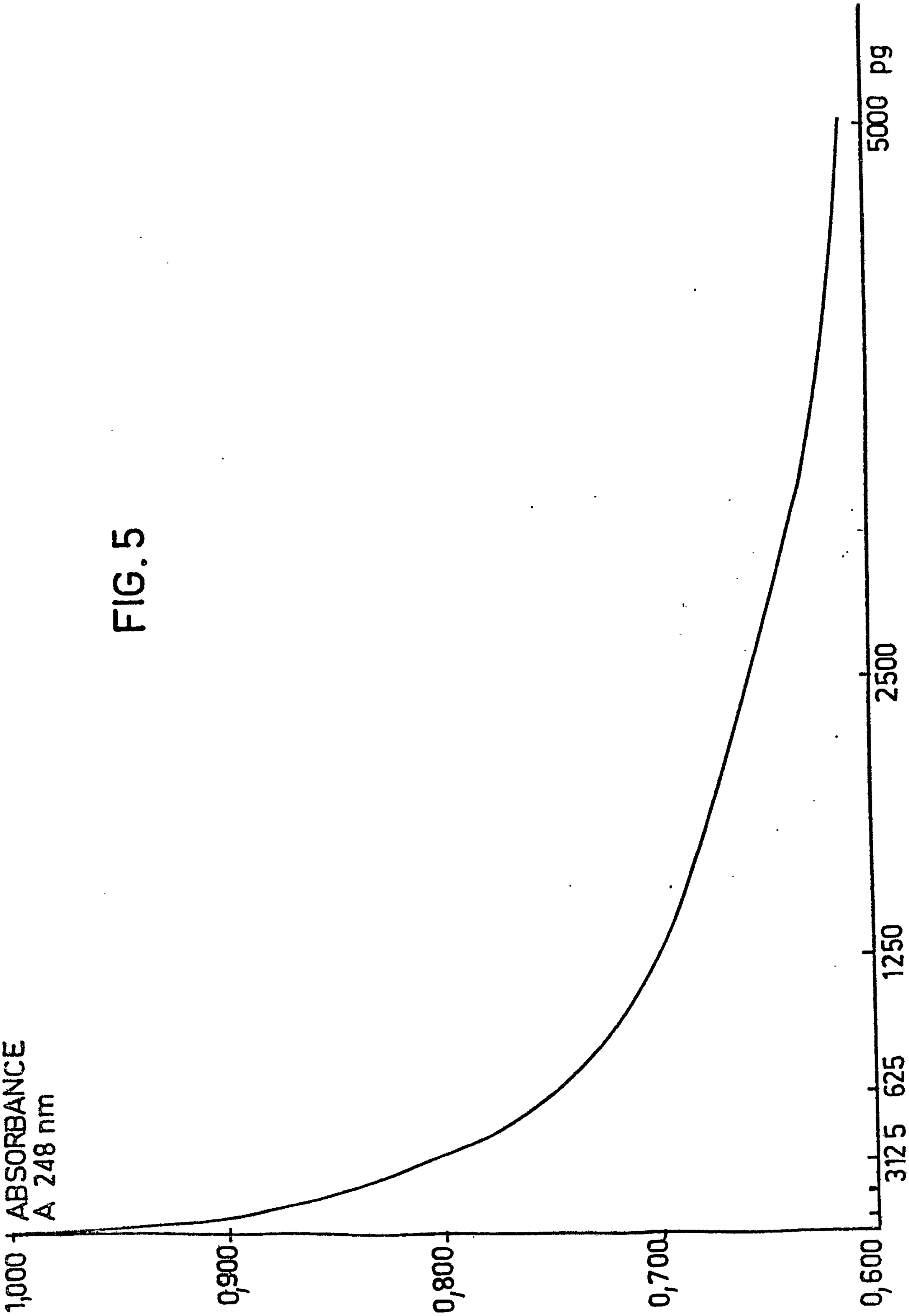
ABSORBANCE
A 248 nm
1,000
0,900
0,800
0,700
0,600
312 5
625
1250
2500
5000 pg

FIG. 5

FIG.6